Europäisches Patentamt

European Patent Office

Office européen des brevets

(11) Publication number: **0 307 847 B1**

## EUROPEAN PATENT SPECIFICATION

(45) Date of publication of patent specification: **02.12.92**   (51) Int. Cl.⁵: **A61K 37/547**

(21) Application number: **88114903.3**

(22) Date of filing: **13.09.88**

The file contains technical information submitted after the application was filed and not included in this specification

(54) Thrombolytic agent.

(30) Priority: **18.09.87 GB 8721951**

(43) Date of publication of application:
**22.03.89 Bulletin 89/12**

(45) Publication of the grant of the patent:
**02.12.92 Bulletin 92/49**

(84) Designated Contracting States:
**AT BE CH DE ES FR GB GR IT LI LU NL SE**

(56) References cited:

**ARCH. MAL. COEUR, vol. 79, no. 4, 1986;
G.FRANCOIS et al., pp. 435-442.**

**ANN. BIOL. CHEM., vol. 43, no. 6, 1985;
M.RONCATO et al., pp. 851-855.**

**THROMBOSIS RESEARCH, vol. 42, 1986, Pergamon Press Ltd. (US); O.MATSUO et al., pp. 187-194.**

**THE JOURNAL OF BIOLOGICAL CHEMISTRY, vol. 261, no. 3, 25 January 1986, The American Society of Biological Chemists, Inc. (US); H.R.LIJNEN et al., pp. 1253-1258.**

**BLOOD, vol. 69, no. 1, January 1987;
R.PANNELL et al., pp. 22-26.**

**BLOOD, vol. 67, no. 5, May 1986; R.PANNELL et al., pp. 1215-1223.**

(73) Proprietor: **THROMBOSIS RESEARCH INSTITUTE
9-11 Fulwood Place Gray's Inn
London WC1V 6HO(GB)**

(72) Inventor: **Kakkar, Vijay Vir
6, Aspen Copse
Bickley Kent BR1 2NZ(GB)**
Inventor: **Scully, Michael Finbarr
Douglas House Crays Hill
Essex CM11 2XN(GB)**
Inventor: **Ellis, Vincent John
79 Lincoln Road
London E7 8ON(GB)**
Inventor: **Watahiki, Yoichi
922 Omiya-cho Naka-gun
Ibaraki-ken(JP)**

(74) Representative: **Armitage, Ian Michael et al
MEWBURN ELLIS 2 Cursitor Street
London EC4A 1BO(GB)**

Rank Xerox (UK) Business Services

## Description

The invention relates to the therapeutic lysis of fibrin blood clots in human patients.

Several naturally-occurring enzymes are known to participate in the lysis of fibrin clots, and have been used therapeutically to lyse clots in patients, e.g. coronary patients, in whom life-threatening clots have formed. Two of these enzymes are pro-urokinase (pro-UK) and urokinase (UK). UK is believed to be synthesized in vivo in a single-chain, zymogenic form (pro-UK) which is converted to the two-chain form (UK) by a proteolytic cleavage. An alternative name for pro-UK is single chain urinary plasminogen activator (scu-PA).

Pro-UK can be used for thrombolysis in early myocardial infarction in order to limit or even prevent definite myocardial necrosis. However, when pro-UK is used alone, clot lysis proceeds only after a lag time during which little or no lysis occurs, and relatively high dosages, of above 6,500,000 I.U. are necessary in order to obtain good results. Pro-urokinase, either as such or after conversion to the two-chain form (UK) is a plasminogen activator; that is, it converts inactive plasminogen to plasmin, a proteolytic enzyme which degrades the fibrin which is a major constituent of blood clots.

The native form of plasminogen occurring in human plasma is a 92 kD single-chain protein with an amino-terminal glutamic acid residue (glu-plasminogen). This native form can be converted to a lower molecular weight form by the limited proteolytic action of plasmin, with the loss of an 8 kD peptide and the exposure of an amino-terminal lysine residue (lys-plasminogen or lys-plg). Lys-plasminogen has a higher affinity for fibrin than glu-plasminogen, and is also activated more rapidly by two-chain urokinase. It has also recently been found that lys-plasminogen is activated by pro-UK more effectively than is glu-plasminogen.

We have now found that blood clots are lysed more rapidly by pro-UK in an in vitro model when lys-plasminogen is present, whereas glu-plasminogen has no effect. The presence of a sulphated polysaccharide such as heparin or chondroitin sulphate may also have a beneficial effect on clot lysis.

Accordingly, the present invention provides a method of lysing fibrin blood clots in patients in need of such treatment comprising concomitant administration of effective amounts of lys-plasminogen and of pro-UK.

Alternatively, the invention provides the use of lys-plasminogen together with pro-UK, in free or fixed combination, as a thrombolytic agent. As a further alternative, the invention provides the use of lys-plasminogen for the preparation of an agent for the potentiation of the thrombolytic effect of pro-UK.

Pro-UK may be obtained from urine as originally described in European Patent 40238, or from culture of natural cell lines or cell lines transformed by recombinant DNA technology, and may be obtained in forms differing in degree of glycosylation or in other respects.

The pro-UK used according to this invention will normally be human pro-UK having the amino-acid sequence as shown for pro-UK in Figure A of European Patent Application 92182, ignoring the lead sequence from -20 to -1. However, the pro-UK used in the invention may vary from this structure by substitution, deletion or addition at one or more amino acid residues, so long as it retains essentially the same biological activity. Thus for example a non-human pro-UK or a compound such as described in PCT patent application WO 86/04351, having a different amino acid substituted for lys-135 and/or phe-157 or as described in European Patent Application 200 451, having substitutions or deletions at phe-157 or lys-158 is within the definition of "pro-UK" so long as it retains biological activity. Furthermore, truncated forms of pro-UK for example such as described by Rijken et al in Thrombosis Research 42 749-760 and 761-768, are included in the definition of "pro-UK" so long as biological activity is retained. Such molecules may have their amino-terminal at lys-136, corresponding to single-chain LMW-UK, or at other convenient sites for example ala-132, lys-144 and glu-150. Forms of pro-UK having additional amino acids for example an initial methionine are also included if active.

Plasminogen prepared from human plasma or Cohn fraction III in the absence of plasmin inhibition contains varying amounts of glu-and lys-plasminogen (but principally lys-plg). Conversion to all lys-plg may be achieved by incubation with plasmin as described by Claeys et al Thromb. Res. 3 315 (1973), or with elastase. Lys-plg is also obtainable commercially, e.t. from Kabi Co.

Patients receiving particular benefit from the invention include patients with DVT or pulmonary embolism, patients who have recently suffered myocardial infarction resulting from clots and those with arterial thrombosis resulting in peripheral ischemia. Pro-UK and lys-plg, separately or together, are admixed with a pharmaceutically acceptable carrier substance, e.g. saline, and administered parenterally, either intravenously or by injection into affected arteries or the heart. Intravenous administration, which is preferred, may be by infusion, by a bolus injection, or by a combination of these.

Preferably, the patient also receives heparin, for example a bolus injection of 5000 I.U. heparin before administration of any PUK or lys-plg, optionally followed by infusion of 1000 I.U./hr of heparin. The heparin may also be given in the form of a mixture with the pro-UK or the lys-plg, or as a mixture of all three components. Heparin may be replaced by other sulphated polysaccharides for example chondroitin sulphate K.

The preferred quantity of lys-plg used in the process according to the invention is up to 30 mg, preferably 15-30 mg. The preferred quantity of pro-UK used is less than 6 500,000 I.U., more preferably from 2 000,000 I.U. to 4 000,000 I.U.

The above quantity of lys-plg is most effective when administered as a bolus injection prior to administration of pro-UK. However, it may be preferred, for ease of administration, to administer a mixture of pro-UK and lys-plg in a single injection or infusion, preferably as a single bolus injection. According to a further aspect of the invention, therefore, there is provided a pharmaceutical composition comprising a mixture of lys-plg and pro-UK together with a pharmaceutically acceptable diluent or carrier, or a mixture of lys-plg and pro-UK in pure lyophilised form.

The components according to the invention may also be presented in twin-pack form, that is, as a single package containing separate unit dosages of lys-plg and pro-UK with instructions for concomitant administration.

The quantity of pro-UK is expressed herein either by weight (mg) or in International Units (IU) based on the International Reference Preparation of UK assayed on a standard fibrin plate (Brakman, Fibrinolysis, A Standardized Fibrin Plate Method and a Fibrinolytic Assay of Plasminogen, Scheltema & Holkema NV, Amsterdam, 1967, pp. 1-24). Pro-UK is assayed after activation by plasmin as described in: Gurewich et al., J. Clin. Invest. (1984) 73, pp. 1731-1739. Pro-UK has a specific activity of about 100 000 IU/mg.

The weight ratio of lys-plg to pro-UK in the composition according to the invention is preferably from 1:13 to 3:2, more preferably 3:8 to 3:2 particularly 1:2 to 1:1. Preferably the composition is made up in unit dosage forms e.g. vials for injection or bottles for infusion, each containing 15 - 30 mg of lys-plg and 2,000,000 - 4,000,000 I.U. of pro-UK.

According to a further aspect of the invention, the patient is given a bolus injection of lys-plg and a bolus injection of pro-UK followed by an infusion of pro-UK. The amount of lys-plg given as a bolus is as described above, i.e. preferably 15 - 30 mg. The amount of pro-UK given as a bolus is preferably 300,000 - 1,000,000 I.U., more preferably 400,000 - 600,000 I.U., particularly 500,000 I.U. These quantities may be administered as two separate bolus injections, delivered in any order, or from a twin-barrelled syringe; or as a single bolus injection of a mixture of lys-plg and PUK, which represents a further composition according to the invention.

The weight ratio of lys-plg to pro-UK in this composition is preferably from 3:2 to 10:1, more preferably 2.5:1 to 7.5:1. Preferably, however, the lys-plg is administered before any pro-UK is given.

It may also be desirable to administer heparin together with the bolus of lys-plg and/or pro-UK. The amount of heparin to be added to the above quantities of lys-plg, pro-UK or lys-plg/pro-UK mixture is preferably from 1000 to 10,000 I.U., more preferably about 5000 I.U.

The amount of pro-UK given as a subsequent infusion is preferably approximately 100,000 I.U./minute, which may be given for approx. 40 min., i.e. a total of 4,000,000 I.U. In many cases opening of the occluded artery may occur within 30 minutes or even less, and infusion may be stopped after this time if arterial opening is established for example by coronary angiography.

For use in this aspect of the invention, there is provided a kit comprising a unit dosage of lys-plg for injection, a unit dose of pro-UK for injection, and a unit dose of pro-UK for infusion. The kit is preferably presented in a single package, and in association with instructions for administration. The unit dosage forms for injection may be sterile solutions of lys-plg or pro-UK in pure water or in physiological saline, or may be in lyophilised or freeze dried solid form to which sterile water or saline is added before injection. The unit dosage form of pro-UK for infusion may be a solution in physiological saline or other sterile aqueous medium for infusion, or a lyophilised or freeze dried solid or a liquid concentrate from which an infusion solution can be made up. The solution for injection or infusion may contain other components, for example buffer salts such as $Na_2HPO_4/NaH_2PO_4$ and preservatives e.g. mannitol and human albumin, and these components may also be present in the lyophilised or freeze dried solid forms.

The quantity of lys-plg and of pro-UK in the unit dosage forms for injection is preferably as described above, particularly 15 - 30 mg of lys-plg and 500,000 I.U. of pro-UK; the quantity of pro-UK in the unit dosage form for infusion is preferably 3,000,000 - 5,000,000 I.U. more preferably 4,000,000 I.U.

It has also been found that the addition of small amounts of urokinase to the combination of lys-plg and pro-UK may increase the rate of clot lysis even further, without severe systemic fibrinolysis. For example, either high- or low-molecular weight urokinase (HMW-UK or LMW-UK) may be added in an amount sufficient to give 10-50 I.U./ml of serum, i.e. a dose of approximately 30,000 - 150,000 I.U. for a normal adult. The UK may be administered before the pro-UK and lys-plg, simultaneously with them as a fixed three-component combination, or, preferably, immediately thereafter.

Example 1:

**Methods**

200 ul aliquots of human plasma plus $^{125}$I-fibrinogen were clotted with thrombin (3u/ml) in the presence of 5mM CaCl$_2$. These clots were then incubated in 2ml plasma in the presence of pro-urokinase (200 und 300 I.U./ml), with the addition of unfractionated heparin (0,1,10 ug/ml) and lys-plasminogen (0,30,100% of plasma concentration). Clot lysis was determined by the release of radioactivity from the clot into the supernatant plasma.

**Results**

At a pro-urokinase concentration of 200 I.U./ml, in the absence of lys-plasminogen, heparin at concentrations of up to 10 ug/ml had little effect on plasma clot lysis, with 50% lysis being observed after $3^1/2$ - $4^1/2$ hours, after an initial lag of approx. $1^1/2$-2 hours. Data obtained at a pro-urokinase concentration of 300 I.U./ml also showed no effect of heparin with 50 % lysis in $2^1/4$ hours after a 1 hour lag phase.

When varying concentrations of lys-plasminogen were incubated with 200 I.U./ml pro-urokinase in the plasma clot lysis system, a very significant acceleration of clot lysis was observed. At a lys-plasminogen concentration 30% that of plasma plasminogen concentration, the lag phase of clot lysis was reduced to 30 minutes, and the 50% lysis time reduced to 2 hours. With 100% lys-plasminogen, the 50 % lysis time was slightly shortened, but now the lag phase was virtually abolished. Very similar results were obtained at 300 I.U./ml pro-urokinase, with the 50 % lysis time reduced from $2^1/4$ hours to $1^1/2$ hours by both 30 and 100 % lys-plasminogen, and 100 % lys-plasminogen completely abolishing the lag phase of clot lysis.

Glu-plasminogen added to identical concentrations in these experiments had no effect on clot lysis.

Example 2:

The same method for in vitro study of clot lysis is used as in Example 1. In this example, a combination of lys-plasminogen, pro-urokinase and LMW-urokinase (Abbokinase) was studied.

**Results**

In plasma containing 100 I.U./ml of pro-UK, additional lys-plg (20 % of normal plasma concentration) and low levels of urokinase (10-50 I.U./ml), it was observed that rapid and effective lysis of the clot occurred without severe lowering of systemic fibrinogen levels.

Example 3: Dosage Forms

a) Mixture for injection
15 mg lys-plg and 3,000,000 I.U. pro-UK are dissolved in 20 ml sterile isotonic saline and packaged in a vial for injection.
b) Lyophilized mixtures
A solution of 30 mg lys-plg and 3,000,000 I.U. pro-UK is lyophilized and the solid residue sealed in a vial to which saline for injection can be added.
c) Twin-barrelled syringe
One barrel of the prepackaged syringe contains 20 mg lys-plg in 10 ml sterile isotonic saline, the other contains 3,000,000 I.U. pro-UK in 10 ml sterile isotonic saline.
d) Twin-pack
A twin-pack comprises one vial containing 30 mg lys-plg in lyophilized form and one bottle containing 3,000,000 I.U. pro-UK in lyophilized form with instructions to add a small quantity of sterile saline to the lys-plg for injection and a larger quantity of sterile saline to the pro-UK for infusion.

**Claims**

1. In free or fixed combination, lys-plasminogen together with pro-UK, for use as a thrombolytic agent.

2. The use of lys-plasminogen for the preparation of an agent for the potentiation of the thrombolytic effect of pro-UK.

3. The combination or use according to Claim 1 or Claim 2 in which the dosage of lys-plasminogen is up to 30 mg and the dosage of pro-UK is up to 650,000 I.U.

4. The combination or use according to Claim 3 in which the dosage of lys-plg is 15-30 mg and the dosage of pro-UK is from 2,000,000 I.U. to 4,000,000 I.U.

5. A twin-pack containing separate unit dosages of lys-plasminogen and pro-UK with instructions for concomitant administration.

6. A kit comprising a unit dosage of lys-plg for injection, a unit dose of pro-UK for injection, and a unit dose of pro-UK for infusion.

7. A kit according to Claim 6 in which the unit dosage of lys-plg is 15-30 mg, the unit dosage of pro-UK for injection is from 400,000 - 600,000 I.U. and the unit dosage of pro-UK for infusion is 3,000,000 I.U. - 5,000,000 I.U.

8. A twin-pack or kit according to any one of Claims 5-7 in which at least one of the dosage forms of lys-plg or pro-UK also contains from 1,000 to 10,000 I.U. heparin.

9. A pharmaceutical composition comprising a mixture of lys-plg and pro-UK together with a pharmaceutically acceptable diluent or carrier, or a mixture of lys-plg and pro-UK in pure lyophilized form.

10. A pharmaceutical composition according to Claim 9 in which the weight ratio of lys-plg to pro-UK is from 1:13 to 3:2.

11. A pharmaceutical composition according to Claim 9 in which the weight ratio of lys-plg to pro-UK is from 3:2 to 10:1.

12. A pharmaceutical composition according to Claim 9 also comprising heparin or chondroitin sulphate.

13. A pharmaceutical composition according to Claim 9 or Clam 10 also comprising urokinase.

**Patentansprüche**

1. Lys-Plasminogen gemeinsam mit pro-UK in freier oder fixer Kombination zur Verwendung als ein thrombolytisches Agens.

2. Verwendung von lys-Plasminogen zur Herstellung eines Agens zum Potenzieren der thrombolytischen Wirkung von pro-UK.

3. Kombination oder Verwendung nach Anspruch 1 bzw. 2, worin die Dosis von lys-Plasminogen bis zu 30 mg beträgt und die Dosis von pro-UK bis zu 6 500 000 I.E. beträgt.

4. Kombination oder Verwendung nach Anspruch 3, worin die Dosis von lys-plg 15-30 mg beträgt und die Dosis von pro-UK von 2 000 000 I.E. bis 4 000 000 I.E. beträgt.

5. Zwillingspackung, die getrennte Einheitsdosen von lys-Plasminogen und pro-UK mit Anweisungen zur gleichzeitigen Verabreichung enthält.

6. Set, umfassend eine Einheitsdosis von lys-plg zur Injektion, eine Einheitsdosis von pro-UK zur Injektion und eine Einheitsdosis von pro-UK zur Infusion.

7. Set nach Anspruch 6, worin die Einheitsdosis von lys-plg 15-30 mg beträgt, die Einheitsdosis von pro-UK zur Injektion von 400 000 - 600 000 I.E. beträgt und die Einheitsdosis von pro-UK zur Infusion 3 000 000 I.E. - 5 000 000 I.E. beträgt.

8. Zwillingspackung oder Set nach einem der Ansprüche 5-7, worin zumindest eine der Dosierungsformen von lys-plg oder pro-UK auch von 1 000 bis 10 000 I.E. Heparin enthält.

9. Pharmazeutische Zusammensetzung, umfassend eine Mischung von lys-plg und pro-UK gemeinsam mit einem pharmazeutisch verträglichen Verdünnungsmittel oder Träger, oder eine Mischung aus lys-plg und pro-UK in reiner lyophilisierter Form.

10. Pharmazeutische Zusammensetzung nach Anspruch 9, worin das Gewichtsverhältnis zwischen lys-plg und pro-UK von 1:13 bis 3:2 beträgt.

11. Pharmazeutische Zusammensetzung nach Anspruch 9, worin das Gewichtsverhältnis zwischen lys-plg und pro-UK von 3:2 bis 10:1 beträgt.

12. Pharmazeutische Zusammensetzung nach Anspruch 9, die auch Heparin oder Chondroitinsulfat umfaßt.

13. Pharmazeutische Zusammensetzung nach Anspruch 9 oder 10, die auch Urokinase umfaßt.

**Revendications**

1. En combinaison libre ou fixée, lys-plasminogène avec pro-UK pour une utilisation comme agent thrombolytique.

2. Utilisation de lys-plasminogène pour la préparation d'un agent pour la potentialisation de l'effet thrombolytique de pro-UK.

3. Combinaison ou utilisation selon la revendication 1 ou la revendication 2, où la dose de lys-plasminogène peut atteindre 30 mg et la dose de pro-UK atteint 650.000 U.I.

4. Combinaison ou utilisation selon la revendication 3, où la dose de lys-plg est de 15-50 mg et la dose de pro-UK est comprise entre 2.000.000 U.I. et 4.000 000 U.I.

5. Emballage double contenant des doses unitaires séparées de lys-plasminogène et pro-UK avec les instruction pour une administration concomitante.

6. Nécessaire comprenant une dose unitaire de lys-plg pour injection, une dose unitaire de pro-UK pour injection et une dose unitaire de pro-UK pour infusion.

7. Nécessaire selon la revendication 6, où la dose unitaire de lys-plg est de 15-30 mg, la dose unitaire de pro-UK pour injection est de 400.000 à 600.000 U.I. et la dose unitaire de pro-UK pour infusion est de 3.000.000 à 5.000.000 U.I.

8. Emballage ou nécessaire selon l'une quelconque des revendications 5-7 où au moins l'une des formes de dose de lys-plg ou pro-UK contient également 1.000 à 10.000 U.I. d'héparine.

9. Composition pharmaceutique comprenant un mélange de lys-plg et de pro-UK avec un diluant ou support acceptable en pharmacie ou bien un mélange de lys-plg ou pro-UK sous une forme lyophilisée pure.

10. Composition pharmaceutique selon la revendication 9, où le rapport pondéral de lys-plg à pro-UK est compris entre 1:13 et 3:2.

11. Composition pharmaceutique selon la revendication 9, où le rapport pondéral de lys-plg à pro-UK est compris entre 3:2 et 10:1.

12. Composition pharmaceutique selon la revendication 9, contenant également du sulfate d'héparine ou de chondroïtine.

13. Composition pharmaceutique selon la revendication 9 ou la revendication 10, contenant également de l'urokinase.